# EUROPEAN PATENT APPLICATION

(11) **EP 4 775 155 A1**
(43) Date of publication of application: **15.07.2026**
(21) Application number: 26151072.1
(22) Date of filing: 09.01.2026
(51) Int. Cl.: A61B 17/04, A61B 17/06

(54) **TENSIONABLE TETHER**

(30) Priority: 14.01.2025 US 202563744910 P; 06.01.2026 US 202619440768
(71) Applicant: Wright Medical Technology, Inc., Memphis, TN 38117 (US)
(72) Inventor: BATEMAN, Cody Keith, Oakland, 38060 (US); SMIETANA, Michael, Memphis, 38104 (US)
(74) Representative: Zimmermann & Partner Patentanwälte mbB

(57) **Abstract**

A tensionable loop of a suture strand, includes the suture strand including a first end and a second end, wherein the first end includes an eyelet of the suture strand, and the second end is passed through the eyelet, encircled 180° back to a butt of the eyelet, and pierced through two points on the suture strand adjacent to the butt of the eyelet such that exerting a force on the second end tightens the tensionable loop and exerting a force on a portion of the suture strand before a first point in which the suture is pierced through itself loosens the tensionable loop.

## Description

### CROSS-REFERENCE TO RELATED APPLICATIONS

This application claims benefit to U.S. Provisional Application No. 63/744,910, filed January 14, 2025, the disclosure of which is incorporated herein by reference in its entirety.

### BACKGROUND

Various injuries include separation of soft tissue from one or more bones and/or separation of bones from normally anatomical correct positioning. Maintaining the bones in the correct anatomical positions during healing is important to provide proper soft tissue reattachment and proper bone healing. In one example, during syndesmosis repair, a first bone and a second bone must be maintained in a fixed position to allow the connective tissue to refuse.

Current suture systems include a suture anchor and one or more knots for maintaining sutures in a fixed position under tension. Most often the suture length needed to create a bone-to-bone augment is estimated prior to anchoring. Knots formed in the sutures can cause irritation during healing and can be subject to tearing due to friction or other forces applied to the knot. Current systems further require surgeons to form knots during surgery. Such systems are prone to failure and increase time of surgery.

Just as importantly, once a suture knot has been tied, it usually is very difficult to subsequently adjust the tension (i.e., the level of tensile force, which can also be called tightness, tautness, or similar terms) in that suture strand. Most augments on the market do not allow for tension adjustment after anchors have been fixated. However, there are numerous situations in which a surgeon may wish to adjust, tighten, or loosen a plurality of suture strands between anchors.

### SUMMARY OF THE DISCLOSURE

To overcome the problems described above, embodiments of the present disclosure including a tensionable loop of a suture strand including the suture strand including a first end and a second end, wherein the first end includes an eyelet of the suture strand, and the second end is passed through the eyelet, encircled 180° back to a butt of the eyelet, and pierced through two points on the suture strand adjacent to the butt of the eyelet such that exerting a force on the second end tightens the tensionable loop and exerting a force on a portion of the suture strand before a first point in which the suture is pierced through itself loosens the tensionable loop.

In an aspect, the tensionable loop is attached between at least two anchors to provide adjustable tension between the at least two anchors.

In an aspect, the suture strand is made of Ultra-High Molecular Weight Polyethylene (UHMWPE).

In another embodiment, a surgical system for soft tissue fixation includes a plurality of bone anchors; a plurality of suture strands with one each attached to a respective one of the plurality of bone anchors to define suture rings; and a suture that passes through itself to define a tensionable loop having adjustable tension and that passes through the suture rings.

In an aspect, the plurality of bone anchors each include a cannulated body having a central passageway and a proximal end and a distal end, and the plurality of suture strands pass through the central passageway to attach to the respective one of the plurality of bone anchors to define the suture ring.

In an aspect, the plurality of bone anchors each include a cannulated body having a central passageway and a proximal end and a distal end, the surgical system further comprises a plurality of inserts, and each of the plurality of suture strands passes through an eyelet of a respective one of the plurality of inserts before each of the plurality of inserts is passed through a central passageway of a respective one of the plurality of bone anchors to attach the insert and the suture strand at the distal end the bone anchor.

In an aspect, the plurality of bone anchors each include an eyelet at a distal end, and the plurality of suture strands pass through the eyelet to attach to the respective one of the plurality of bone anchors to define the suture ring.

In an aspect, a length of each of the suture rings is adjustable, e.g., by use of at least one of a sliding-knot ring, a cinch sleeve, and a buckle/cleat.

In an aspect, the tensionable loop is attached to each of the plurality of bone anchors to provide adjustable tension between the plurality of bone anchors.

In an aspect, the tensionable loop includes the suture having a first end and a second end, the first end includes an eyelet of the suture strand, and the second end is passed through the eyelet, encircled about 180° back to a butt of the eyelet, and pierced through two points on the suture adjacent to the butt of the eyelet such that exerting a force on the second end tightens the tensionable loop and exerting a force on a portion of the suture before a first point in which the suture is pierced through itself loosens the tensionable loop.

In an aspect, exerting a force on a free end of the suture tightens the tensionable loop and exerting a force on a portion of the suture before a first point in which the suture is pierced through itself loosens the tensionable loop.

In another embodiment, a method of adjusting tension between bone portions includes drilling a first hole in a first bone portion and a second hole in a second bone portion; attaching a suture loop to each of at least two bone anchors; inserting one of the at least two bone anchors into each of the first hole and the second hole; creating a tensionable loop with a suture; attaching the suture loops of the at least two bone anchors to the tensionable loop; and tightening or loosening any of the suture loops or the tensionable loop to adjust tension between the at least two bone anchors.

In an aspect, attaching the suture loop to each of the at least two bone anchors includes feeding a suture strand through an eyelet of an insert and locking the insert to a respective one of the at least two bone anchors.

In an aspect, attaching the suture loop to each of the at least two bone anchors includes feeding a suture strand through a respective one of the at least two bone anchors.

In an aspect, attaching the suture loop to each of the at least two bone anchors includes feeding a suture strand through an eyelet at a distal end of a respective one of the at least two bone anchors.

In an aspect, creating the tensionable loop includes providing the suture having a first end and a second end, creating an eyelet at the first end, and passing the second end through the eyelet, encircling the second end about 180° back to a butt of the eyelet, and piercing the second end through two points on the suture adjacent to the butt of the eyelet.

The method can further include adjusting the tension between the at least two bone anchors using the tensionable loop by exerting a force on a free end of the suture to tighten the tensionable loop and exerting a force on a portion of the suture before a first point in which the suture is pierced through itself to loosen the tensionable loop.

Further disclosed herein is a tensionable loop of a suture strand that includes the suture strand including a first end and a second end, wherein the first end includes an eyelet of the suture strand, and the second end is passed through the eyelet, encircled 180° back to a butt of the eyelet, and pierced through two points on the suture strand adjacent to the butt of the eyelet such that exerting a force on the second end tightens the tensionable loop and exerting a force on a portion of the suture strand before a first point in which the suture is pierced through itself loosens the tensionable loop.

The above and other features, elements, characteristics, steps, and advantages of the present invention will become more apparent from the following detailed description of preferred embodiments of the present invention with reference to the attached drawings.

### BRIEF DESCRIPTION OF THE DRAWINGS

FIGS. 1A to 1C show a knotless tensionable tether, according to an embodiment.
FIG. 2 shows a surgical system having a knotless construct.
FIGS. 3A to 3F are used to show and describe steps for assembling an insert to an anchor.
FIGS. 4A to 4F illustrate steps of a method of using a knotless tensionable tether, according to an embodiment.
FIGS. 5A and 5B illustrate loosening tension of a tensionable tether.
FIGS. 6A and 6B show a knotted tensionable tether, according to an embodiment.
FIG. 7 shows components of a tensionable tether having a knotted configuration similar to that shown and described with respect to FIG. 6.
FIGS. 8A to 8I illustrate steps of a method of using a knotted tensionable tether, according to an embodiment.
FIGS. 9A to 9G show a tensionable tether used in an ankle repair application.
FIG. 10 is a close-up view of features of a tensionable loop.

### DETAILED DESCRIPTION

Reference will now be made in detail to embodiments of the present disclosure, examples of which are illustrated in the accompanying drawings. Whenever possible, the same reference numerals will be used throughout the drawings to refer to the same or like parts. However, this disclosure may be embodied in many different forms and should not be construed as limited to the embodiments set forth herein.

In the following description, reference is made to the accompanying drawings that form a part thereof, and in which is shown by way of illustrating specific exemplary embodiments in which the disclosure may be practiced. These embodiments are described in sufficient detail to enable those skilled in the art to practice the concepts disclosed herein, and it is to be understood that modifications to the various disclosed embodiments may be made, and other embodiments may be utilized, without departing from the scope of the present disclosure. The following detailed description is, therefore, not to be taken in a limiting sense.

Directional terms as used herein - for example up, down, right, left, front, back, top, bottom, vertical, horizontal - are made only with reference to the figures as drawn and are not intended to imply absolute orientation.

Unless otherwise expressly stated, it is in no way intended that any method set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred, in any respect. This holds for any possible non-express basis for interpretation, including: matters of logic with respect to arrangement of steps, operational flow, order of components, or orientation of components; plain meaning derived from grammatical organization or punctuation, and; the number or type of embodiments described in the specification.

As used herein, the singular forms "a," "an," and "the" include plural references unless the context clearly dictates otherwise. Thus, for example, reference to "a" component includes aspects having two or more such components, unless the context clearly indicates otherwise.

Disclosed is a tensionable tether that permits tissue or bone anchors and a tensionable suture loop to be fully fixated in place. The surgeon can easily tighten or loosen the tensionable tether incrementally to the desired tension. The surgeon can also remove all instruments from the field and use both hands to articulate an anatomical joint or simulate movement to test current tension and can then continue to adjust, tighten, or loosen the tether as desired. Disclosed embodiments allow for incremental tightening or loosening after full fixation of the anchors and suture. No specialized, suture material, anchors, nor instruments are required. The suture type can be braided with a round cross section or flat-braided with a rectangular cross section such as suture tape. The suture material may be Ultra-High Molecular Weight Polyethylene (UHMWPE), as is commonly used, e.g., suture tape flat-braided rectangular cross-section or round or flat-braided/tape forms. Additionally, other suture materials such as, polyester, PLLA, blends may be employed with adequate results.

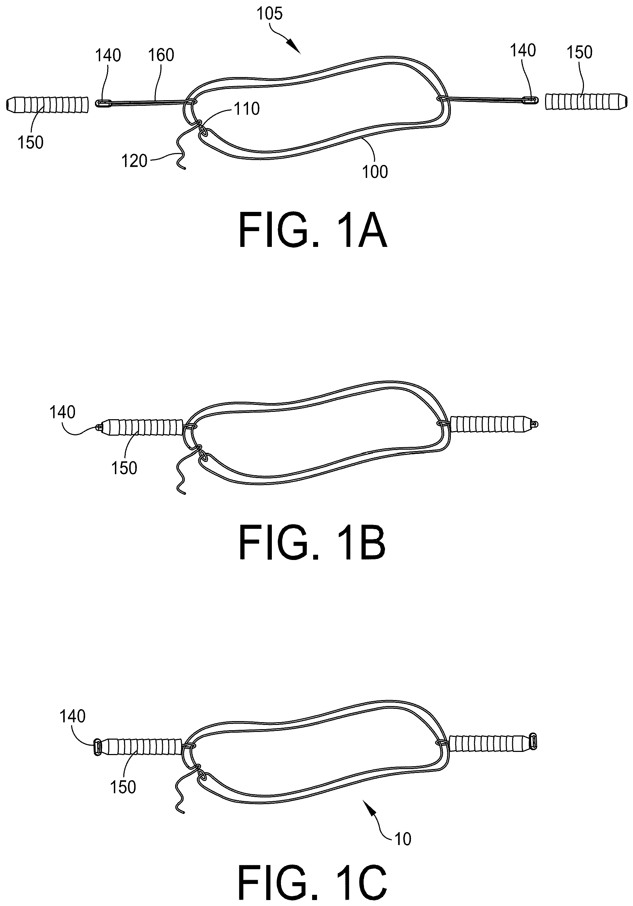

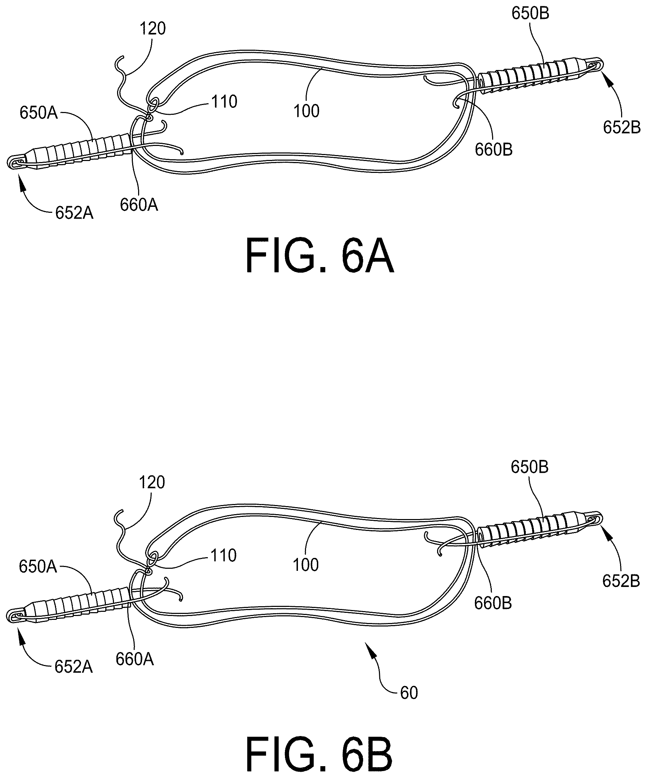

Disclosed embodiments include a tensionable loop of a suture and its integration with anchors using either a knotless (e.g., FIG. 1) or knotted (e.g., FIG. 6) configuration to define a tensionable tether. The tensionable tether can be either tightened or loosened by the surgeon. By design, the tensionable tether holds its tension across the joint and may only be tightened or loosened if force is applied to the free-end. Embodiments of tensionable tether are shown described as including two bone anchors, but it should be appreciated that more than two bone anchors can be included in some applications.

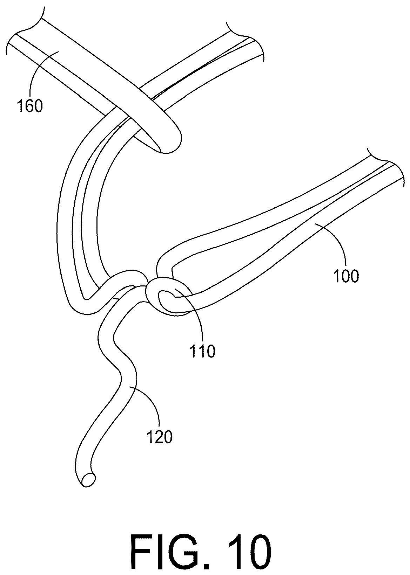

As shown in FIGS. 1 and 6, for example, a tensionable loop 105 can be tied by starting with a suture 100 with an eyelet 110 on one side. A free-end 120 of the suture 100 can be passed through the eyelet 110 and encircled 180° back to the beginning, i.e., the region of the eyelet 110 where the strand re-joins or approaches the standing part of the suture, hereinafter referred to as "butt of the eyelet" 110. The free-end 120 can be pierced through two or more points on the suture 100 strand very close to the butt of the eyelet 110. This creates the tensionable loop 105. FIGS. 1 and 6 shown that the suture construct with tensionable loop 105 can be attached to two anchors to provide adjustable tension between the anchors. FIG. 10 is a close-up view of the eyelet 110 and the suture 100 passed through itself.

FIGS. 1A-1C show a knotless configuration of a tensionable tether 10 where an insert 140 of an anchor 150 can be connected to the tensionable loop 105 using a suture ring 160. As shown, the insert 140 can be oval or elliptical shaped. Suture rings 160 can be used to allow for the tensionable loop 105 to slide freely between fixation points provided by the anchors 150 during tightening or loosening. The insert 140 can be locked into a cannulated anchor 150 by pressing it axially through the anchor 150, as shown in FIG. 1B, and then rotating the insert 140 to prevent it from backing out, as shown in FIG. 1C that shows the tensionable tether 10.

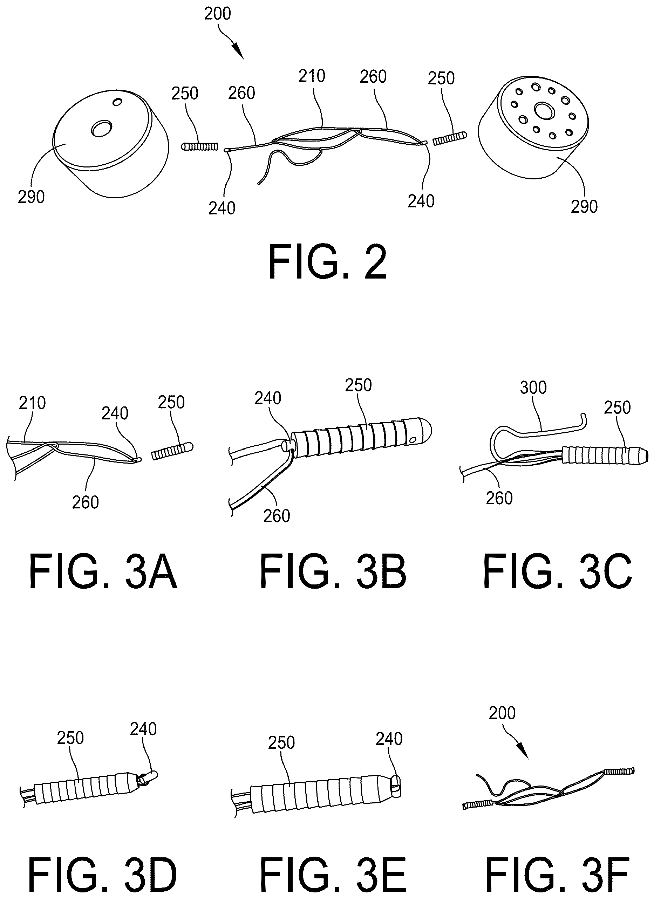

FIG. 2 shows a tensionable tether 200 for soft tissue fixation having a knotless construct similar to that shown and described with respect to FIG. 1. The construct can include a tensionable loop 210 made from a suture, anchors 250 having a cannulated body, and inserts 240 attached to a suture ring 260. FIG. 2 also includes foam blocks 290 that simulate bones as the tensionable tether created is designed to reinforce ligament or bone fixation. The anchors 250 can have a cannulated body with a central passageway and a proximal end and a distal end. The inserts 240 can have a proximal end and a distal end that is sized to be put through the central passageway of the cannulated body of the anchors 250. The tensionable loop 210 can be coupled to the proximal end of the inserts 240 via the suture rings 260. The tensionable loop 210 defined by the suture can include a flexible strand that extends through the suture rings 260 attached to the inserts 240. Lengths of the suture rings 260 can be adjusted to allow a surgeon to control tension of the tensionable tether 200. The foam blocks 290 can include holes sized to fit the anchors 250 and simulate being a bone and used for development and training.

FIGS. 3A to 3F are used to show and describe steps for assembling an insert 240 to an anchor 250. FIG. 3A shows the tensionable loop 210 tied through the suture ring 260 that is attached to the insert 240 which has not been joined to the anchor 250. For assembly, the insert 240 is inserted into an opening of the cannulated body of the anchor 250, as shown in FIG. 3B. In FIG. 3C, the insert (not visible) is being pushed through the cannulated body of the anchor 250 using a tool or instrument 300 until the insert 240 is completely through the anchor 250, like that shown in FIG. 3D. Once through the anchor 250, the oval shaped insert 240 is turned so the long axis is perpendicular to a longitudinal axis along the length of the cannulated anchor 250 to prevent the insert 240 from being pulled back out. Performing these steps with two inserts 240 and two anchors 250 completes assembly with the suture 210 to provide the tensionable tether 200 shown in FIG. 3F. The anchors 250 can then be inserted into bone to create fixation across a joint or two bone portions.

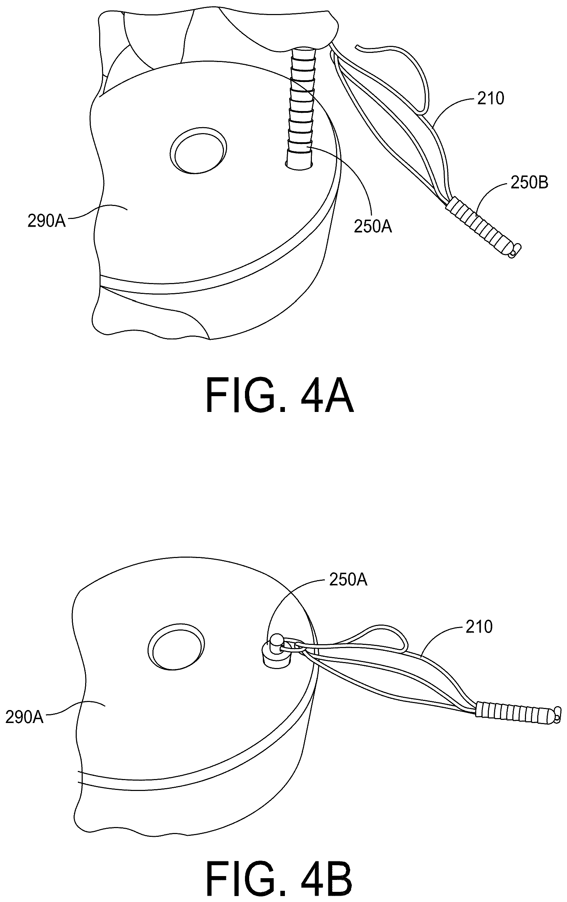

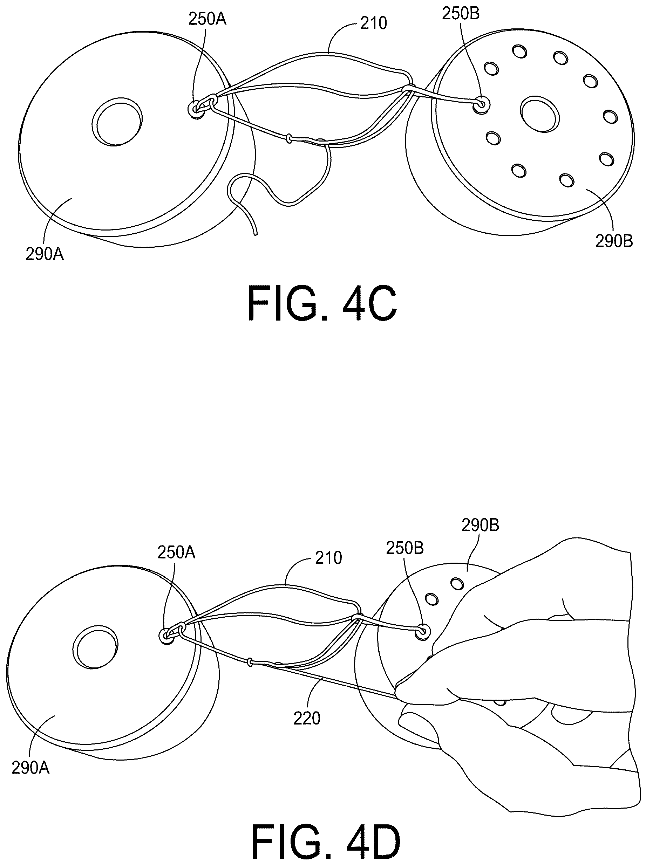

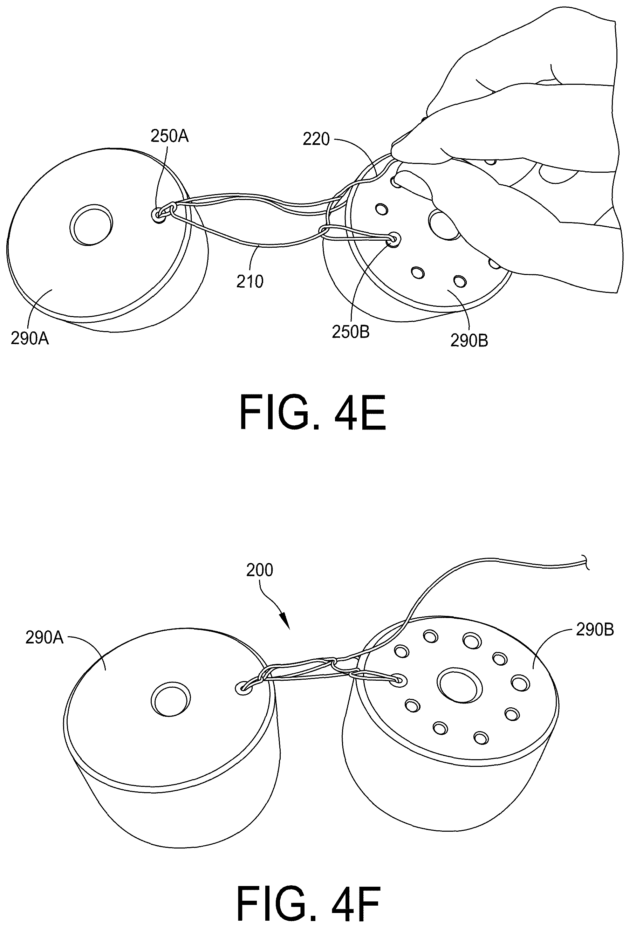

According to some embodiments, a method of using the knotless tensionable tether 200 is shown with respect to FIGS. 4, 5, and 9. Referring to FIG. 4, a hole can be drilled in a first bone, simulated by the foam block 290A, and a first anchor 250A can be inserted into the hole, as shown in FIGS. 4A and 4B. Likewise, a second hole can be drilled in a second bone, simulated by the foam block 290B, and a second anchor 250B can be inserted into the hole, as shown in FIG. 4C. Tension of the tensionable tether 200 can be tightened by pulling a loose strand 220 of the tensionable loop 210, as shown in FIGS. 4D and 4E. FIG. 4F shows the result of a tightened tensionable tether 200 between the foam blocks 290A and 290B.

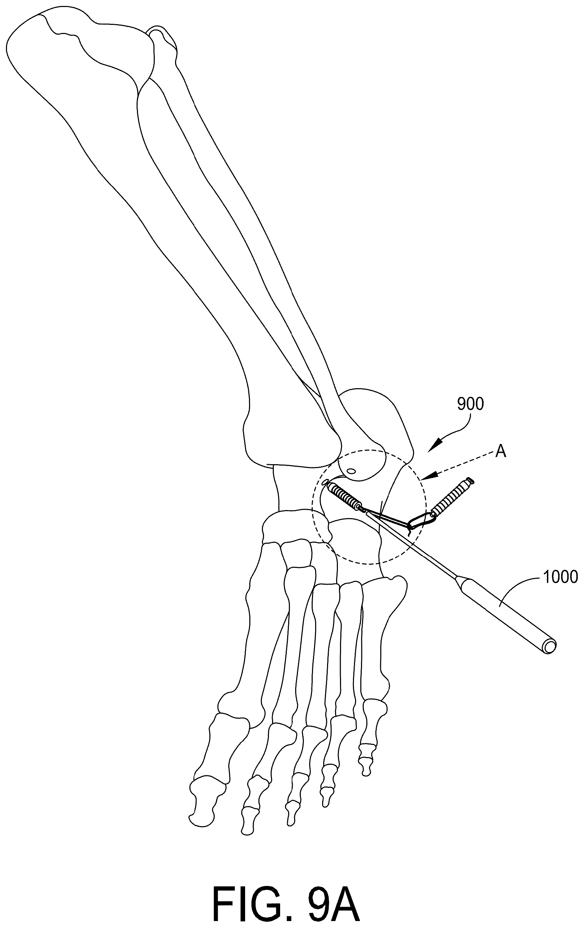

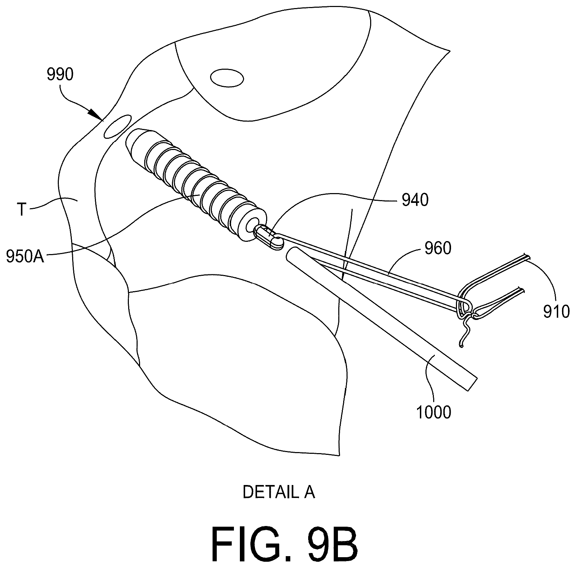

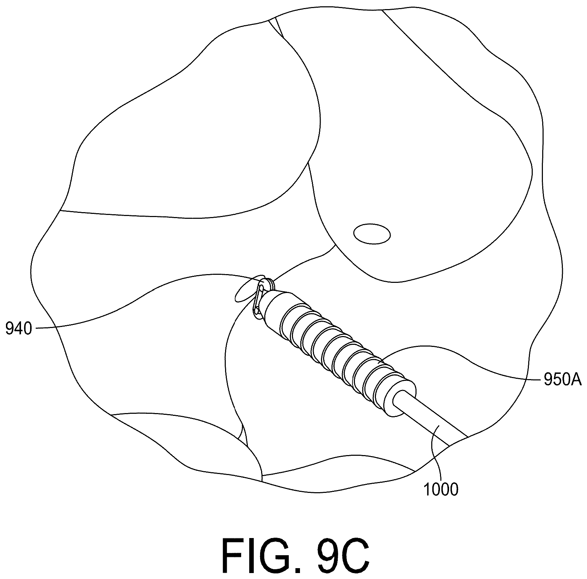

FIGS. 9A to 9G show a tensionable tether 900 used in an ankle repair application. FIG. 9A is an exploded view of the tensionable tether 900 and an instrument 1000. FIG. 9B is a close up of detail view A from FIG. 9A showing a first anchor 950A, an insert 940, a suture ring 960, and a tensionable loop 910 of a suture prior to implantation into a talus T. An instrument 1000 is in line to aid with threading the oval insert 940 through the first anchor 950A and locking it into place in a hole 990 in the talus T. FIG. 9C is a close-up view of the instrument 1000 used to lock the insert 940 into place in the first anchor 950A.

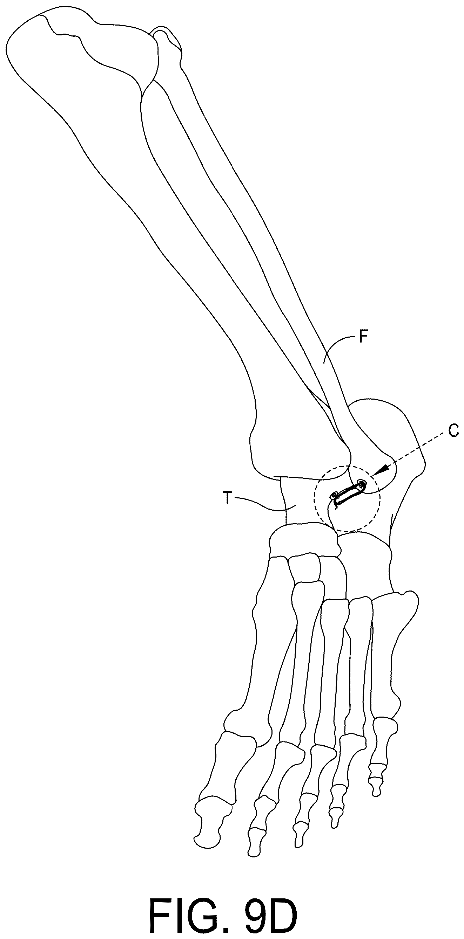

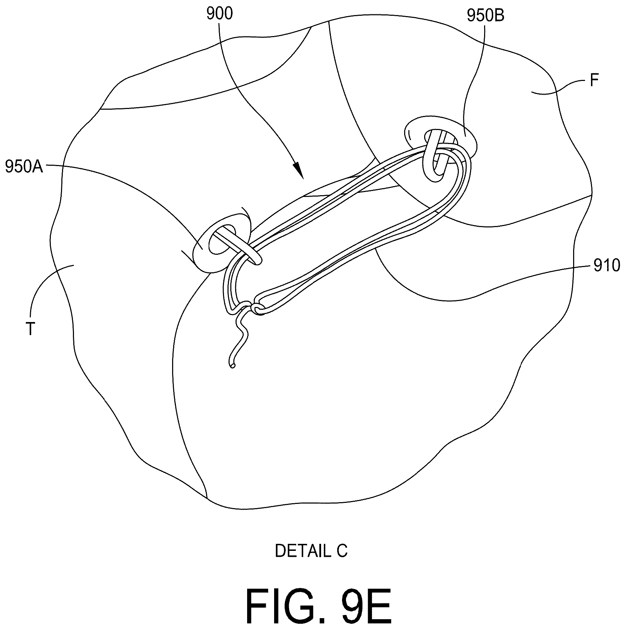

FIG. 9D is a view of the tensionable tether 900 after both the first anchor 950A and a second anchor 950B have been implanted, one in the talus T and one in a fibula F. FIG. 9E is a close up of detail C from FIG. 9D showing the tensionable tether 900 in place. Note that the tensionable tether 900 can be tightened or loosened as desired.

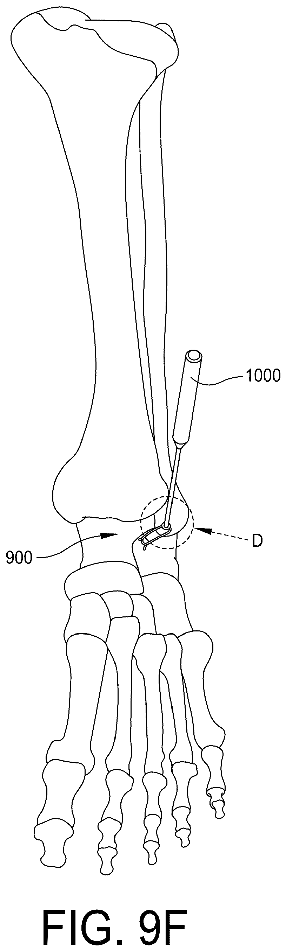

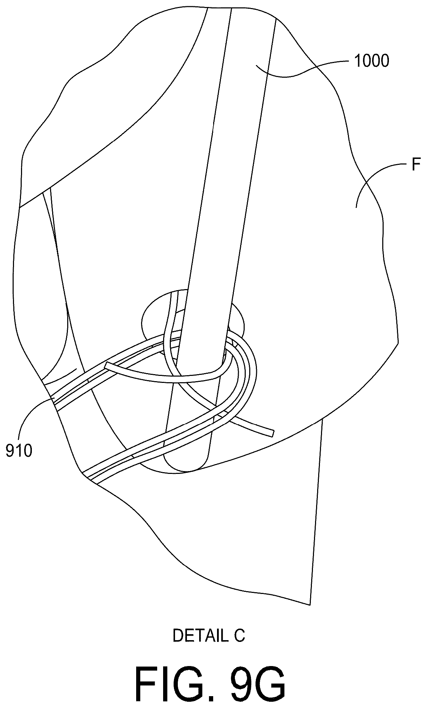

FIG. 9F is a view showing that the instrument 1000 can be used as a spacer to complete the knotted configuration. FIG. 9G is a close-up of detail D of FIG. 9F. FIG. 9G shows that the instrument 1000 can be used to create space and/or hold the suture 910 in place while a knot is formed in the tensionable tether 900.

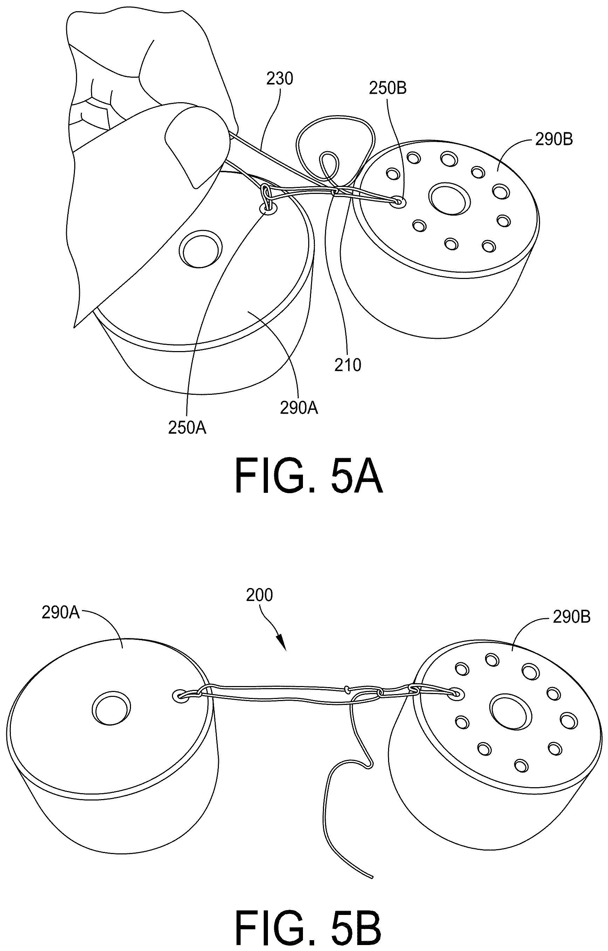

Referring to FIG. 5, FIG. 5A shows that tension of the tensionable tether 200 can be loosened by pulling on a portion 230 of the tensionable loop 210 of the suture construct. FIG. 5B shows the result of a loosened tensionable tether 200 between the foam blocks 290A and 290B.

As previously mentioned, FIG. 6 illustrates a knotted configuration of a tensionable tether 60, according to some embodiments. FIGS. 6A and 6B show a knotted configuration where anchors 650A, 650B can be connected to the tensionable loop 100 using respective suture rings 660A, 660B. As shown, the suture rings 660A, 660B can be created by a surgeon during surgery by passing a suture through an eye 652A, 652B at a distal end of the anchors 650A, 650B and tying a knot above the anchors 650A, 650B. The suture rings 660A, 660B can be used to allow for the tensionable loop 100 to slide freely between fixation points provided by the anchors 650A, 650B during tightening or loosening. FIG. 6A shows the suture rings 660A, 660B with the suture passed through the eyes 652A, 652B and FIG. 6B shows the suture rings 660A, 660B completed with knots represented by the crossed ends of the suture 120.

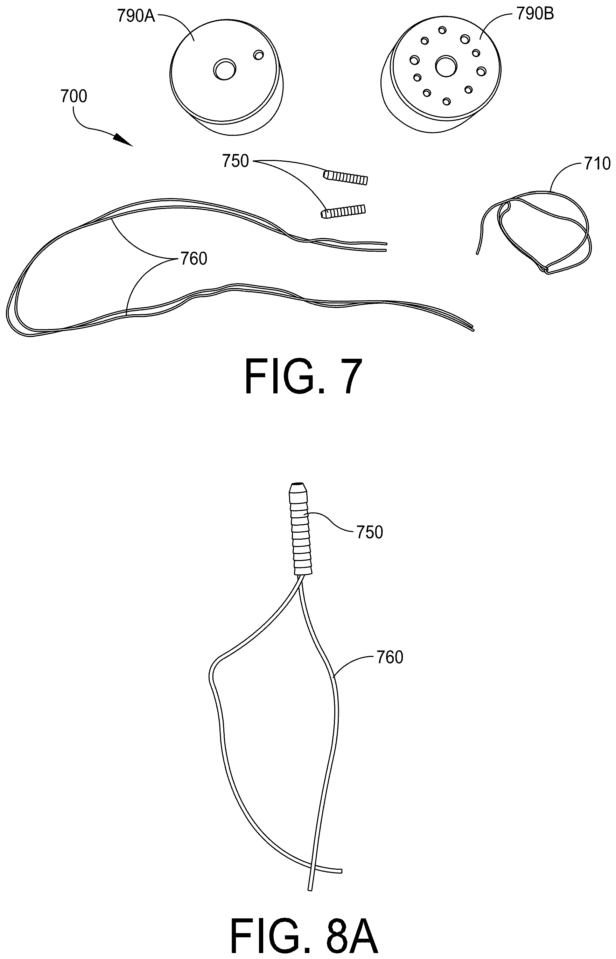

FIG. 7 shows components of a surgical tensionable tether 700 for soft tissue fixation having a knotted construct similar to that shown and described with respect to FIG. 6. As shown, the tensionable tether 700 can include a tensionable loop 710, anchors 750, and sutures for suture ring 760. FIG. 7 also shows foam blocks 790A, 790B that simulate bones as the tensionable tether 700 is designed to reinforce ligaments or bone fixation. The anchors 750 can have an eyelet at their distal end. The tensionable loop 710 can be coupled to the anchors 750 via a suture ring 760. The tensionable construct defined by the suture loop 710 can include a flexible strand that extends through the suture ring 760. The suture rings 760 can project outwardly a distance from their respective anchors 750 to allow a surgeon to control tension of the tensionable tether 700. The foam blocks 790 can include holes sized to fit the anchors 750 and simulate being a bone for development and training.

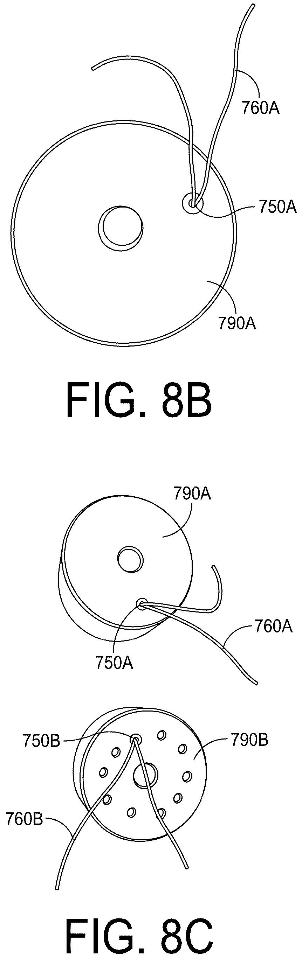

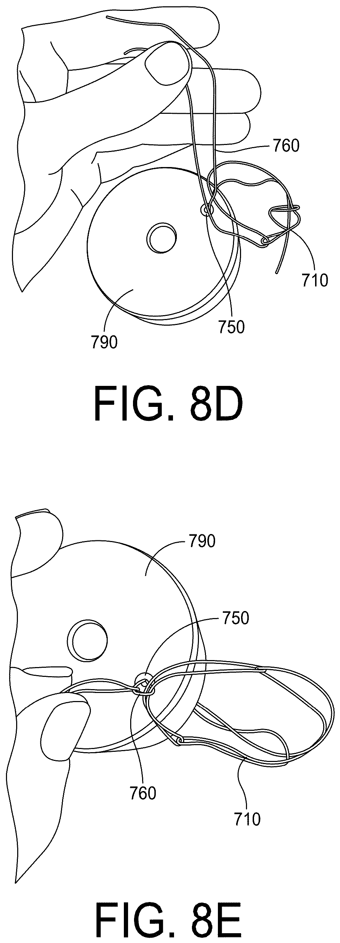

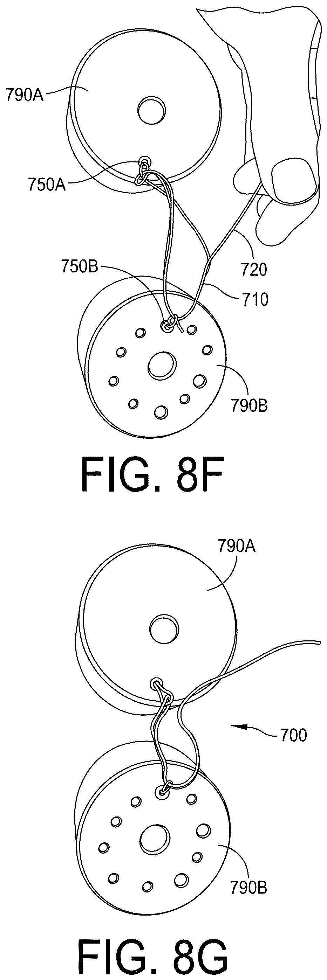

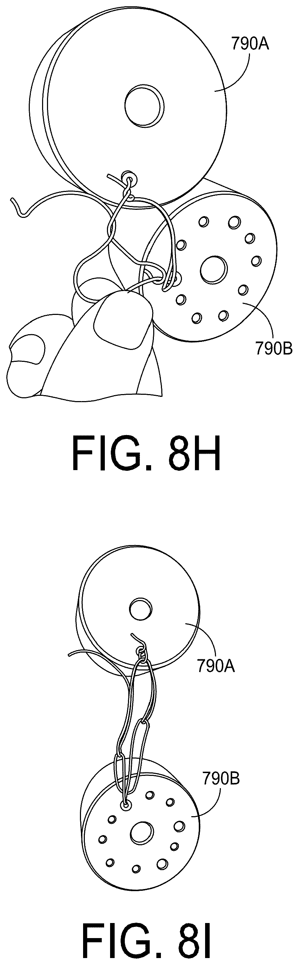

According to some embodiments, steps of a method of using the knotted tensionable tether system 700 is shown with respect to FIGS. 8A to 8I. FIG. 8A shows that a suture for the suture ring 760 can be fed through an anchor 750, for example through an eyelet at the distal end, like that shown in FIG. 6, or through a cannulated opening. The anchors 750 can be inserted into predrilled holes in bones (simulated by the foam blocks 790A, 790B), as shown in FIGS. 8B and 8C. FIG. 8D shows that one end of the suture forming the suture rings 760 can be fed through the tensionable loop 710 and then tied off with the other end of the suture to form the suture ring 760 around the tensionable loop 710, as shown in FIG. 8E. FIG. 8F shows that tension of the tensionable tether can be tightened by pulling a loose strand 720 of the tensionable loop 710. FIG. 8G shows the result of a tightened tensionable tether 700 between the foam blocks 790A and 790B.

It should be understood that the foregoing description is only illustrative of the present invention. Various alternatives and modifications can be devised by those skilled in the art without departing from the present invention. Accordingly, the present invention is intended to embrace all such alternatives, modifications, and variances that fall within the scope of the appended claims.

Further disclosed herein is the subject-matter of the following clauses:
1. A tensionable loop of a suture strand, comprising:
   the suture strand including a first end and a second end, wherein the first end includes an eyelet formed in the suture strand, and the second end is passed through the eyelet, encircled about 180° back to a butt of the eyelet, and pierced through two points on the suture strand adjacent to the butt of the eyelet such that exerting a force on the second end tightens the tensionable loop and exerting a force on a portion of the suture strand before a first point at which the suture strand is pierced through itself loosens the tensionable loop.
2. The tensionable loop of clause 1, wherein the suture strand comprises Ultra-High Molecular Weight Polyethylene.
3. The tensionable loop of clause 1 or of any of the preceding clauses, wherein the suture strand is braided with a round cross section.
4. The tensionable loop of clause 1 or of any of clauses 1-2, wherein the suture strand is flat-braided with a rectangular cross section.
5. The tensionable loop of clause 1 or of any of the preceding clauses, wherein the second end is pierced through more than two points on the suture strand adjacent to the butt of the eyelet.
6. The tensionable loop of clause 1 or of any of the preceding clauses, wherein the tensionable loop is configured to hold tension absent force applied to the second end and to be selectively tightened or loosened in response to force applied to the suture strand at predetermined locations.
7. The tensionable loop of clause 1 or of any of the preceding clauses, further comprising at least two bone anchors and at least one suture ring, wherein the tensionable loop extends through the at least one suture ring that is attached to at least one of the at least two bone anchors to provide adjustable tension between the at least two bone anchors.
8. The tensionable loop of clause 7, wherein a length of the at least one suture ring is adjustable to control tension transfer between the tensionable loop and the at least two bone anchors.
9. The tensionable loop of clause 7 or of any of clauses 7-8, wherein the at least one suture ring is defined by a suture extending through an eyelet at a distal end of at least one of the at least two bone anchors and tied to form a loop.
10. The tensionable loop of clause 7 or of any of clauses 7-9, wherein the at least one suture ring is defined by a suture passing through an eyelet of an insert and the insert is locked within a cannulated body of at least one of the at least two bone anchors.
11. The tensionable loop of clause 1 or of any of the preceding clauses, wherein the eyelet at the first end is integral to the suture strand.
12. The tensionable loop of clause 1 or of any of the preceding clauses, wherein the eyelet at the first end is formed by splicing the suture strand into itself.
13. A surgical system for fixation, comprising:
   a plurality of bone anchors; a plurality of suture rings each attached to a respective one of the plurality of bone anchors; and a suture that passes through itself to define a tensionable loop having adjustable tension and that passes through the plurality of suture rings.
14. The surgical system of clause 13, wherein each of the plurality of bone anchors comprises a cannulated body having a central passageway, a proximal end, and a distal end, and wherein each suture ring is coupled to a respective insert having an eyelet, the insert being sized to pass axially through the central passageway and to be locked at the distal end of the cannulated body.
15. The surgical system of clause 14, wherein each insert is rotatable after passage through the central passageway to orient a long axis of the insert transverse to a longitudinal axis of the cannulated body to resist back-out.
16. The surgical system of clause 13 or of any of clauses 13-15, wherein at least one of the plurality of bone anchors includes an eyelet at a distal end and the corresponding suture ring is formed by passing a suture through the eyelet and tying the suture to form a loop.
17. The surgical system of clause 13 or of any of clauses 13-16, wherein the tensionable loop is configured to be tightened by pulling a free end of the suture and to be loosened by pulling a portion of the suture before a first piercing point of the suture passing through itself.
18. The surgical system of clause 13 or of any of clauses 13-17, wherein the plurality of bone anchors are dimensioned for implantation in predrilled holes in respective bones.
19. The surgical system of clause 13 or of any of clauses 13-18, wherein the tensionable loop is configured to slide relative to the plurality of suture rings to equalize tension between the plurality of bone anchors during tightening and loosening.
20. The surgical system of clause 13 or of any of clauses 13-19, wherein the suture defining the tensionable loop comprises UHMWPE.
21. The surgical system of clause 13 or of any of clauses 13-20, wherein the suture rings project outwardly from their respective bone anchors to facilitate tension adjustment of the tensionable loop.
22. The surgical system of clause 13 or of any of clauses 13-21, wherein each suture ring has an adjustable length to allow incremental tension control of the tensionable loop between the plurality of bone anchors.
23. A method of adjusting tension between bone portions, comprising:
   drilling a first hole in a first bone portion and a second hole in a second bone portion; attaching a suture loop to each of at least two bone anchors;
   inserting one of the at least two bone anchors into each of the first hole and the second hole; creating a tensionable loop with a suture;
   attaching the suture loops of the at least two bone anchors to the tensionable loop; and
   tightening or loosening any of the suture loops or the tensionable loop to adjust tension between the at least two bone anchors.
24. The method of clause 23, wherein attaching the suture loop to each of the at least two bone anchors comprises feeding a suture strand through an eyelet of an insert and locking the insert to a respective one of the at least two bone anchors.
25. The method of clause 23, wherein attaching the suture loop to each of the at least two bone anchors comprises feeding a suture strand through a cannulated opening of a respective one of the at least two bone anchors.
26. The method of clause 23, wherein attaching the suture loop to each of the at least two bone anchors comprises feeding a suture strand through an eyelet at a distal end of a respective one of the at least two bone anchors and tying the suture strand to form the suture loop.
27. The method of clause 23, wherein creating the tensionable loop comprises providing the suture having a first end and a second end, creating an eyelet at the first end, passing the second end through the eyelet, encircling the second end 180° back to a butt of the eyelet, and piercing the second end through two points on the suture adjacent to the butt of the eyelet.
28. The method of clause 23, further comprising adjusting the tension between the at least two bone anchors using the tensionable loop by pulling a free end of the suture to tighten the tensionable loop and pulling a portion of the suture before a first point at which the suture is pierced through itself to loosen the tensionable loop.
29. The method of clause 23, wherein tightening or loosening comprises sliding the tensionable loop relative to the suture loops to redistribute tension across a joint between the first bone portion and the second bone portion.
30. The method of clause 23, wherein the tensionable loop is adjusted incrementally after fixation of the at least two bone anchors within the first bone portion and the second bone portion.
31. A tensionable tether, comprising:
   at least two bone anchors configured for implantation in respective bones;
   at least one suture ring attached to at least one of the at least two bone anchors; and
   a suture that passes through itself to define a tensionable loop having adjustable tension, the tensionable loop extending through the at least one suture ring to span between the at least two bone anchors; wherein the suture includes a first end and a second end, the first end including an eyelet of the suture, and the second end being passed through the eyelet, encircled about 180° back to a butt of the eyelet, and pierced through two or more points on the suture adjacent to the butt of the eyelet such that exerting a force on the second end tightens the tensionable loop and exerting a force on a portion of the suture before a first point at which the suture is pierced through itself loosens the tensionable loop.
32. A suture strand with a tensionable loop, wherein:
   the suture strand includes a first end and a second end, wherein the first end includes an eyelet formed in the suture strand, and the second end is passed through the eyelet, encircled about 180° back to a butt of the eyelet, and pierced through two points on the suture strand adjacent to the butt of the eyelet to form the tensionable loop such that exerting a force on the second end tightens the tensionable loop and exerting a force on a portion of the suture strand before a first point at which the suture strand is pierced through itself loosens the tensionable loop.
33. The suture strand of clause 32, wherein the suture strand comprises Ultra-High Molecular Weight Polyethylene.
34. The suture strand of any of the preceding clauses 32-33, wherein the suture strand is braided with a round cross section, or wherein the suture strand is flat-braided with a rectangular cross section.
35. The suture strand of any of the preceding clauses 32-34, wherein the second end is pierced through more than two points on the suture strand adjacent to the butt of the eyelet.
36. The suture strand of any of the preceding clauses 32-35, wherein the tensionable loop is configured to hold tension absent force applied to the second end and to be selectively tightened or loosened in response to force applied to the suture strand at predetermined locations.
37. The suture strand of any of the preceding clauses 32-36, further comprising at least two bone anchors and at least one suture ring, wherein the tensionable loop extends through the at least one suture ring that is attached to at least one of the at least two bone anchors to provide adjustable tension between the at least two bone anchors.
38. The suture strand of clause 37, wherein a length of the at least one suture ring is adjustable to control tension transfer between the tensionable loop and the at least two bone anchors, and/or wherein the at least one suture ring is defined by a suture extending through an eyelet at a distal end of at least one of the at least two bone anchors and tied to form a loop, and/or wherein the at least one suture ring is defined by a suture passing through an eyelet of an insert and the insert is locked within a cannulated body of at least one of the at least two bone anchors.
39. The suture strand of any of the preceding clauses 32-38, wherein the eyelet at the first end is integral to the suture strand, and/or wherein the eyelet at the first end is formed by splicing the suture strand into itself.
40. A surgical system for fixation, comprising:
   a plurality of bone anchors;
   a plurality of suture rings each attached to a respective one of the plurality of bone anchors; and
   the suture strand of any of the preceding clauses 32-39 that passes through itself to define the tensionable loop having adjustable tension and that passes through the plurality of suture rings.
41. The surgical system of clause 40, wherein each of the plurality of bone anchors comprises a cannulated body having a central passageway, a proximal end, and a distal end, and wherein each suture ring is coupled to a respective insert having an eyelet, the insert being sized to pass axially through the central passageway and to be locked at the distal end of the cannulated body.
42. The surgical system of clause 41, wherein each insert is rotatable after passage through the central passageway to orient a long axis of the insert transverse to a longitudinal axis of the cannulated body to resist back-out.
43. The surgical system of any of clauses 40-42, wherein at least one of the plurality of bone anchors includes an eyelet at a distal end and the corresponding suture ring is formed by passing a suture through the eyelet and tying the suture to form a loop, and/or wherein the tensionable loop is configured to be tightened by pulling a free end of the suture and to be loosened by pulling a portion of the suture before a first piercing point of the suture passing through itself.
44. The surgical system of any of clauses 40-43, wherein the plurality of bone anchors are dimensioned for implantation in predrilled holes in respective bones, and/or wherein the tensionable loop is configured to slide relative to the plurality of suture rings to equalize tension between the plurality of bone anchors during tightening and loosening.
45. The surgical system of any of clauses 40-44, wherein the suture defining the tensionable loop comprises UHMWPE, and/or wherein the suture rings project outwardly from their respective bone anchors to facilitate tension adjustment of the tensionable loop, and/or wherein each suture ring has an adjustable length to allow incremental tension control of the tensionable loop between the plurality of bone anchors.
46. A tensionable tether, comprising:
   at least two bone anchors configured for implantation in respective bones;
   at least one suture ring attached to at least one of the at least two bone anchors; and
   the suture strand of any of clauses 32-39, wherein the suturte strand passes through itself to define the tensionable loop having adjustable tension, the tensionable loop extending through the at least one suture ring to span between the at least two bone anchors; wherein the suture includes a first end and a second end, the first end including an eyelet of the suture, and the second end being passed through the eyelet, encircled about 180° back to a butt of the eyelet, and pierced through two or more points on the suture adjacent to the butt of the eyelet such that exerting a force on the second end tightens the tensionable loop and exerting a force on a portion of the suture before a first point at which the suture is pierced through itself loosens the tensionable loop.

## Claims

1. A tensionable loop of a suture strand, comprising:
the suture strand including a first end and a second end, wherein the first end includes an eyelet formed in the suture strand, and the second end is passed through the eyelet, encircled about 180° back to a butt of the eyelet, and pierced through two points on the suture strand adjacent to the butt of the eyelet such that exerting a force on the second end tightens the tensionable loop and exerting a force on a portion of the suture strand before a first point at which the suture strand is pierced through itself loosens the tensionable loop.

2. The tensionable loop of claim 1, wherein the suture strand comprises Ultra-High Molecular Weight Polyethylene.

3. The tensionable loop of any of the preceding claims, wherein the suture strand is braided with a round cross section, or wherein the suture strand is flat-braided with a rectangular cross section.

4. The tensionable loop of any of the preceding claims, wherein the second end is pierced through more than two points on the suture strand adjacent to the butt of the eyelet.

5. The tensionable loop of any of the preceding claims, wherein the tensionable loop is configured to hold tension absent force applied to the second end and to be selectively tightened or loosened in response to force applied to the suture strand at predetermined locations.

6. The tensionable loop of any of the preceding claims, further comprising at least two bone anchors and at least one suture ring, wherein the tensionable loop extends through the at least one suture ring that is attached to at least one of the at least two bone anchors to provide adjustable tension between the at least two bone anchors.

7. The tensionable loop of claim 6, wherein a length of the at least one suture ring is adjustable to control tension transfer between the tensionable loop and the at least two bone anchors, and/or wherein the at least one suture ring is defined by a suture extending through an eyelet at a distal end of at least one of the at least two bone anchors and tied to form a loop, and/or wherein the at least one suture ring is defined by a suture passing through an eyelet of an insert and the insert is locked within a cannulated body of at least one of the at least two bone anchors.

8. The tensionable loop of any of the preceding claims, wherein the eyelet at the first end is integral to the suture strand, and/or wherein the eyelet at the first end is formed by splicing the suture strand into itself.

9. A surgical system for fixation, comprising:
a plurality of bone anchors;
a plurality of suture rings each attached to a respective one of the plurality of bone anchors; and
a suture that passes through itself to define a tensionable loop having adjustable tension and that passes through the plurality of suture rings.

10. The surgical system of claim 9, wherein each of the plurality of bone anchors comprises a cannulated body having a central passageway, a proximal end, and a distal end, and wherein each suture ring is coupled to a respective insert having an eyelet, the insert being sized to pass axially through the central passageway and to be locked at the distal end of the cannulated body.

11. The surgical system of claim 10, wherein each insert is rotatable after passage through the central passageway to orient a long axis of the insert transverse to a longitudinal axis of the cannulated body to resist back-out.

12. The surgical system of any of claims 9-11, wherein at least one of the plurality of bone anchors includes an eyelet at a distal end and the corresponding suture ring is formed by passing a suture through the eyelet and tying the suture to form a loop, and/or wherein the tensionable loop is configured to be tightened by pulling a free end of the suture and to be loosened by pulling a portion of the suture before a first piercing point of the suture passing through itself.

13. The surgical system of any of claims 9-12, wherein the plurality of bone anchors are dimensioned for implantation in predrilled holes in respective bones, and/or wherein the tensionable loop is configured to slide relative to the plurality of suture rings to equalize tension between the plurality of bone anchors during tightening and loosening.

14. The surgical system of any of claims 9-13, wherein the suture defining the tensionable loop comprises UHMWPE, and/or wherein the suture rings project outwardly from their respective bone anchors to facilitate tension adjustment of the tensionable loop, and/or wherein each suture ring has an adjustable length to allow incremental tension control of the tensionable loop between the plurality of bone anchors.

15. A tensionable tether, comprising:
at least two bone anchors configured for implantation in respective bones;
at least one suture ring attached to at least one of the at least two bone anchors; and
a suture that passes through itself to define a tensionable loop having adjustable tension, the tensionable loop extending through the at least one suture ring to span between the at least two bone anchors; wherein the suture includes a first end and a second end, the first end including an eyelet of the suture, and the second end being passed through the eyelet, encircled about 180° back to a butt of the eyelet, and pierced through two or more points on the suture adjacent to the butt of the eyelet such that exerting a force on the second end tightens the tensionable loop and exerting a force on a portion of the suture before a first point at which the suture is pierced through itself loosens the tensionable loop.
